# EUROPEAN PATENT APPLICATION

(11) **EP 2 679 169 A1**
(43) Date of publication of application: **01.01.2014**
(21) Application number: 11859495.1
(22) Date of filing: 16.09.2011
(51) Int. Cl.: A61B 17/00

(54) **THREE-DIMENSIONAL RETRACTOR**

(30) Priority: 25.02.2011 JP 2011040943
(71) Applicant: Tokusen Kogyo Co., Ltd, Ono-City, Hyogo 675-1361 (JP); Osaka University, Suita-shi, Osaka 565-0871 (JP)
(72) Inventor: NAKAJIMA, Kiyokazu, Suita-shi Osaka 565-0871 (JP); SHIMIZU, Toshiaki, Ono-shi Hyogo 675-1361 (JP); YAMASHITA, Hiroyuki, Ono-shi Hyogo 675-1361 (JP); YAMAUCHI, Toshiyuki, Ono-shi Hyogo 675-1361 (JP)
(74) Representative: MacLean, Martin Robert
(86) International application number: PCT/JP2011/071205
(87) International publication number: WO 2012/114569

(57) **Abstract**

The present invention provides a retractor that includes an excluding portion having an obtuse distal end, an introduction portion extending from the excluding portion, and a handle portion provided at a proximal end of the introduction portion. The excluding portion and the introduction portion each have an outer diameter that allows insertion into a treatment instrument channel of an endoscope. The excluding portion is configured by a movable wire and a plurality of fixed wires, the movable wire extends through the introduction portion, proximal ends of the fixed wires are fixed to a distal end of the introduction portion, and a distal end of the movable wire and distal ends of the fixed wires are joined to each other. A proximal end of the movable wire is pulled with the handle portion toward the handle portion so that the excluding portion can develop a cocoon shape. The retractor of the present invention does not require an insertion route from the body surface, and, thus, it is useful in minimally-invasive surgery such as laparoscopic surgery or NOTES.

## Description

### [Technical Field]

The present invention relates to a three-dimensional retractor for an endoscope, for excluding organs or the like obstructing an organ that is to be treated or the view during treatment in endoscopic therapy

### [Background Art]

In clinical departments, direct observations and procedures in a body cavity are performed in order to diagnose various disease states, judge the effects of treatment, and determine the policies of treatment, for example. Conventionally, these operations have been performed with a highly invasive approach such as an exploratory laparotomy or an exploratory thoracotomy. However, with the recent widened use of endoscopic surgery, these operations are performed using a laparoscope or a thoracoscope with which the size of incisional wounds is smaller than that in conventional cases.

For example, Patent Documents 1 and 2 disclose trocar systems that, after being inserted from an opening having a small diameter through the skin into a body cavity, can easily expand the through cavity, thereby providing a passage for passing a surgical instrument having a larger diameter. According to these systems, the size of the external wound of a patient is smaller and the time necessary for recovery becomes extremely shorter than those in conventional examples in which a large incision corresponding to the diameter of a surgical instrument is made.

Meanwhile, in order to minimize the size of the external wound of a patient, a new minimally-invasive technique has been developed. This technique is known as Natural Orifice Translumenal Endoscopic Surgery (NOTES: endoscopic surgery with no incision on the body surface), and is a completely new technique that inserts an endoscope from a natural orifice (mouth, anus, vagina, etc.) of a hollow organ into the lumen, makes an incision through the wall of the hollow organ to access a body cavity, and makes a diagnosis or performs procedures or treatment. In theory, this technique needs no incisional wound on the body surface (incisionless), and, thus, this technique is expected to be less invasive than endoscopic surgery. Successful clinical cases of laparoscope-assisted transvaginal or transgastric "hybrid NOTES" are reported in foreign countries, and have been attracting great attention. In near future, "pure NOTES" only with an endoscope, not requiring laparoscope-assist, is expected to be introduced into clinical practice.

Unlike conventional laparotomy surgery in which organs obstructing the surgery can be pushed aside by hand, the above-described endoscopic surgery is problematic in that pushing aside of organs is not easy, and, thus, securing the view optimal for the surgery is difficult. This problem is one factor that makes endoscopic surgery difficult. In order to secure the view and the manipulation space, there is a method that injects gas into a body cavity. However, this method requires general anesthesia, and cannot be said to be minimally-invasive.

In order to alleviate problems regarding the view and the like and to make endoscopic therapy easy, an instrument called retractor has been developed that excludes or draws organs or the like obstructing an organ that is to be treated or the view during treatment. A retractor is required to have a basic function in which, at the time of insertion into the body, an instrument can be inserted through a small opening passage such as a trocar (outer sheath) forming an insertion passage or a small incisional wound. Accordingly, at least at the time of insertion, it is necessary that a retractor has a small diameter (e.g., desirably 10 mm or less in the case of a trocar, and 20 mm or less in the case of a small incisional wound), and is in a rod-like shape. Meanwhile, after insertion into a body cavity, it is required that the shape of the retractor can be changed to have an excluding portion with a certain degree of area size in order to exclude a target safely over a wide area.

In view of these mutually conflicting demands, retractors variously devised have been proposed or been commercially available. For example, there is a retractor in which an excluding portion can spread out like a fan (Patent Document 3, for example). This retractor is such that the fan-like excluding portion is folded and accommodated inside a rod-like tube at the time of insertion of the trocar into the peritoneal cavity, and spreads out like a fan when being pushed out from the rod-like tube in the body cavity. The fan-like excluding portion may spread out to any size with manual operation, or the angle between the excluding portion and the base portion may be changed. These retractors are preferable to exclude the liver and the intestines due to advantages of being capable of excluding an organ over a relatively wide area. Note that the shape is not limited to a fan-like shape, and a large number of various shapes such as a lozenge shape have been proposed.

As a different type, there are snake-like retractors in which a rod-like member of an excluding portion can be bent in a body cavity (Patent Documents 4 and 5, for example). These retractors may be such that the bendable excluding portion is accommodated in a straight shape inside a rod-like tube at the time of insertion of a trocar, and returns to a bent shape when being pushed out from the rod-like tube in the body cavity, or such that the excluding portion in the shape of a rod at the time of insertion can be bended into various shapes in a body cavity and can be kept at the bended shape. These are particularly effective in drawing a target to hold the target in the bent portion, and are widely used in laparoscopic surgery.

Alternatively, there is a retractor having a loop-shaped front end portion (Patent Document 6, for example). This is a type of retractor in which a loop having a small diameter is projected from the front end of the tube, and is used to catch, grasp and draw tissue. This retractor can be inserted through a trocar having a small diameter, and is extremely low invasive, but it cannot be used for exclusion. Meanwhile, as a retractor that excludes and draws an organ or the like inside the body, there is an instrument, including an excluding portion configured by a flat sheet that is made of elastic rubber thin film and a frame that is fixed to the peripheral edge of the flat sheet and substantially surrounds the flat sheet, and a grip portion extending from the excluding portion, wherein the frame is made of superelastic alloy or shape memory alloy, and, thus, the excluding portion can be shaped in a smaller diameter so that this portion can be inserted through a trocar or a small incisional wound at the time of insertion into a body cavity, and can return to an original shape having a large exclusing area in the body cavity (Patent Documents 7 and 8).

Furthermore, as a tool arm that is inserted through the lumen included in a tube for endoscope insertion, a snake-like retractor that is flexible and capable of handling organs and tissue structures is disclosed (Patent Document 9).

All of the above-described retractors have an excluding portion that to develop in a one-dimensional shape (line) or a two-dimensional shape (plane).

Meanwhile, a vital manipulating instrument is disclosed, including a shaft member, a hollow tube rotatably fitted around the shaft member, and elastic filamentary members each having one end fixed to the shaft member and another end fixed to the hollow tube, wherein, when the shaft member and the hollow tube are rotated relative to each other, the elastic filamentary members form an excluding portion (Patent Document 10). This is a three-dimensional retractor that forms a pod-like (cocoon-like) three-dimensional excluding portion, wherein the shape of the excluding portion is controlled by the rotation of the shaft member. Accordingly, the shaft member is required to have a certain degree of diameter size, and, furthermore, since there is a limitation in the force that can be applied to rotate the shaft member, this retractor is not suitable to exclude a large organ such as the liver.

### [Citation List]

### [Patent Literature]

Patent Document 1: US Patent No. 5,320,611
Patent Document 2: Japanese National Publication No. 8-507238
Patent Document 3: Japanese Laid-Open Patent Publication No. 6-154152
Patent Document 4: Japanese National Publication No. 7-502914
Patent Document 5: US Patent Application Publication No. 2002/011536
Patent Document 6: Japanese Laid-Open Patent Publication No. 2000-23989
Patent Document 7: Japanese Laid-Open Patent Publication No. 2003-164459
Patent Document 8: Japanese Laid-Open Patent Publication No. 2005-237398
Patent Document 9: Japanese National Publication No 2007-511247
Patent Document 10: Japanese Laid-Open Patent Publication No. 8-336538

### [Summary of Invention]

### [Technical Problem]

It is an object of the present invention to provide a three-dimensional retractor to be expected in minimally-invasive treatment such as NOTES, that is, a three-dimensional retractor that can freely exclude an organ, and can be inserted into a treatment instrument channel of an endoscope. It is another object thereof to provide a method of securing a view in a surgery in a lumen or a surgery in a body cavity by excluding an organ using this retractor.

### [Solution to Problem]

The inventors have found that, in a retractor configured by an excluding portion, an introduction portion, and a handle portion from the distal end, when the excluding portion is configured by a movable wire and a plurality of fixed wires, the movable wire extends through the introduction portion, proximal ends of the fixed wires are fixed to a distal end of the introduction portion, and a distal end of the movable wire and distal ends of the fixed wires are joined to each other, the excluding portion and the introduction portion can be inserted into a treatment instrument channel of an endoscope, and, when a proximal end of the movable wire is pulled with the handle portion toward the handle portion, the excluding portion can develop a three-dimensional rigid shape, and, thus, completed the present invention.

The present invention provides a retractor comprising an excluding portion having an obtuse distal end, an introduction portion extending from the excluding portion, and a handle portion provided at a proximal end of the introduction portion,
wherein the excluding portion and the introduction portion each have an outer diameter that allows insertion into a treatment instrument channel of an endoscope,
the excluding portion is configured by a movable wire and a plurality of fixed wires,
the movable wire extends through the introduction portion,
proximal ends of the fixed wires are fixed to a distal end of the introduction portion,
a distal end of the movable wire and distal ends of the fixed wires are joined to each other, and
a proximal end of the movable wire is pulled with the handle portion toward the handle portion so that the excluding portion can develop a cocoon shape.

In one embodiment, the movable wire forms a central axis of the cocoon shape.

In one embodiment, the movable wire forms one of the peripheral arcs of the cocoon shape.

In one embodiment, each of the fixed wires that form the peripheral arcs of the cocoon shape has a shaped cross-section.

In one embodiment, each of the fixed wires that form the peripheral arcs of the cocoon shape has a circular arc shaped cross-section.

In one embodiment, each of the fixed wires that form the peripheral arcs of the cocoon shape has a crescent shaped cross-section.

In one embodiment, the excluding portion has a length of 30 to 120 mm.

In one embodiment, each of the fixed wires that form the peripheral arcs of the cocoon shape has a cross-section having a major axis of 0.4 to 1.2 mm.

The present invention also provides a method of excluding an organ, which comprises:
causing an endoscope provided with a treatment instrument channel to be inserted from a natural orifice of a hollow organ into the lumen, or through a wall of the lumen into a body cavity;
inserting the above into the treatment instrument channel;
causing the excluding portion of the retractor to project into the lumen or the body cavity so as to develop a cocoon shape; and
bringing the excluding portion into contact with an inner wall of the hollow organ or an organ in the body cavity, to exclude the inner wall or the organ.

In one embodiment, the above method is performed in surgery selected from the group consisting of laparoscopic surgery and NOTES.

The present invention also provides a method of securing a view in a surgery in a lumen or a surgery in a body cavity, which comprises:
causing an endoscope provided with a treatment instrument channel to be inserted from a natural orifice of a hollow organ into the lumen, or through a wall of the lumen into a body cavity;
inserting the above retractor into the treatment instrument channel;
causing the excluding portion of the retractor to project into the lumen or the body cavity so as to develop a cocoon shape near an inner wall of the hollow organ or an organ in the body cavity, which obstructs the view; and
bringing the excluding portion into contact with the inner wall or the organ, to exclude the inner wall or the organ, thereby securing the view in the lumen or the body cavity.

In one embodiment, the surgery in the body cavity is selected from the group consisting of laparoscopic surgery and NOTES.

### [Advantageous Effects of Invention]

The present invention can provide a three-dimensional retractor that can freely exclude an organ, and can be inserted into a treatment instrument channel of an endoscope. The retractor of the present invention is inserted via a treatment instrument channel of an endoscope into the lumen of a hollow organ or into a body cavity, and quickly develops a cocoon shape with remote manipulation. The developed cocoon shape makes it possible to freely exclude organs. A double-channel endoscope provided with two treatment instrument channels or an overtube provided with a treatment instrument channel can be used so that a surgical device (forceps, electrocautery, etc.) is projected from the remaining one channel, and the front end of the device is guided into the cocoon shape of the retractor. Thus, this retractor is useful in minimally-invasive treatment such as NOTES.

The method of the present invention can secure the view and the manipulation space without injecting gas into a body cavity (gassless surgery), and, thus, general anesthesia is not necessary, and very minimally-invasive endoscopic surgery becomes possible.

### Brief Description of Drawings

[FIG.1] Fig. 1 is a schematic view showing an embodiment of a retractor of the present invention.
[FIG.2] Fig. 2 is a partial schematic view showing an embodiment of an excluding portion of the retractor of the present invention that has been exposed through a treatment instrument channel of an endoscope.
[FIG.3] Fig. 3 shows schematic views showing an embodiment of the excluding portion of the retractor of the present invention.
[FIG.4] Fig. 4 shows schematic views showing an embodiment of the cross-sectional shape of a fixed wire in the retractor of the present invention.
[FIG.5] Fig. 5 shows explanatory views showing an application example in which the retractor of the present invention is applied to colonic polypectomy.
[FIG.6] Fig. 6 shows explanatory views showing an example in which the retractor of the present invention is applied to cholecystectomy.
[FIG.7] Fig. 7 shows explanatory views showing an example in which the retractor of the present invention is applied to closing of a small opening in the stomach in NOTES.

### [Description of Embodiments]

The "endoscope" herein refers to a medical endoscope. Such an endoscope is made of flexible material, and its embedded objective optical system may be configured by fiberglass or CCDs. Furthermore, typically, an optical system for illumination is provided with a light source on the control apparatus side outside the body, guides light via optical fiber, and emits the light from the front end portion, but an LED may be embedded in the front end of the endoscope. Furthermore, the orientation of the front end of the endoscope may be freely changed with remote manipulation. An endoscope having an appropriate size is selected according to a target lumen.

Typically, an endoscope has a treatment instrument channel (sub lumen) in addition to that for the optical system, thereby making it possible to perform local washing, injection of gas or liquid, application of a drug, suction, procedures (grasping, cutting, puncture, etc.) using a dedicated device, and the like. Alternatively, the endoscope has an overtube which may be provided with a treatment instrument channel. Typically, these treatment instrument channels each have an inner diameter of 3 mm.

The "retractor" herein refers to a medical instrument for excluding, pushing aside, drawing, and lifting an obstacle for a target (e.g., an organ) or the view in the medical field. Note that the operations of excluding, pushing aside, drawing, and lifting may be collectively referred to as "retraction" or "retract". The term "exclude" may refer not only to the operation of excluding but also to the operations of pushing aside, drawing, and lifting (that is, it may refer to retraction). It is required that a retractor has an excluding portion which can be changed to have a certain degree of size after being inserted into the body. Examples of the insertion passage include a treatment instrument channel of an endoscope, a trocar (outer sheath), and the like.

The "distal" herein refers to a portion of the instrument farthest from the operator using the instrument, and the term "proximal" refers to a portion of the instrument closest to the operator.

The "cocoon shape" herein refers to a pod shape or an oval spherical shape (rugby ball shape).

Examples of target hollow organs include stomach, small intestine, colon and rectum, vagina, and the like. Examples of other target organs include liver, pancreas, kidney, gall bladder, spleen, womb, lung, and the like.

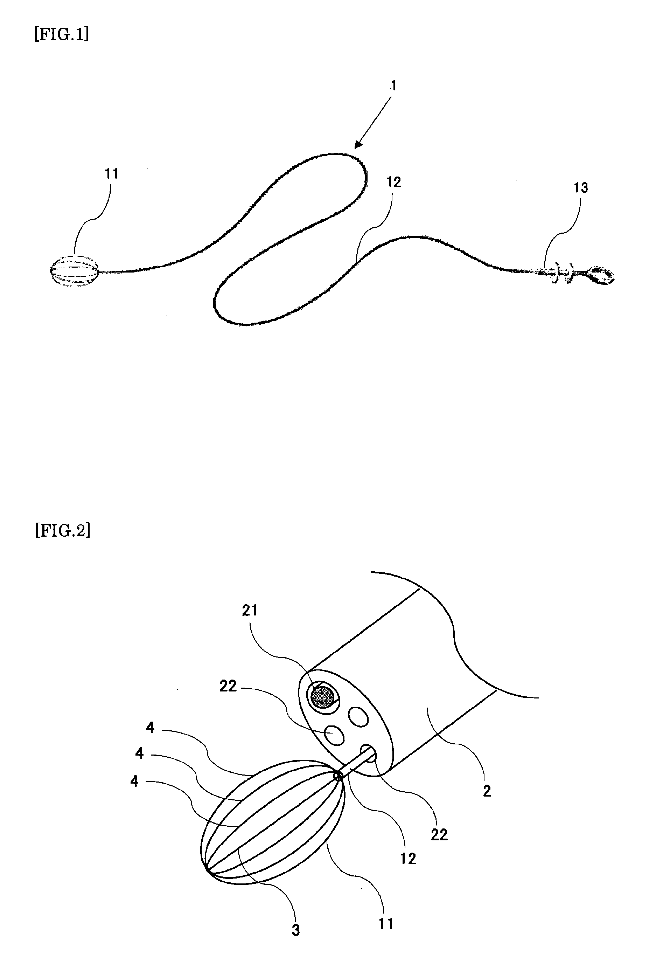

Referring to Fig. 1, a retractor 1 of the present invention includes an excluding portion 11 having an obtuse distal end, an introduction portion 12 extending from the excluding portion 11, and a handle portion 13 provided at a proximal end of the introduction portion 12.

The distal end of the excluding portion 11 has an obtuse shape, that is, a round shape, in order not to damage organs.

The excluding portion 11 and the introduction portion 12 each have an outer diameter that allows these portions to be inserted into treatment instrument channels 22 of an endoscope 2. Since each treatment instrument channels 22 typically has an inner diameter of about 3 mm, the excluding portion 11 and the introduction portion 12 may each have an outer diameter of less than 3 mm when being inserted into the treatment instrument channel 22.

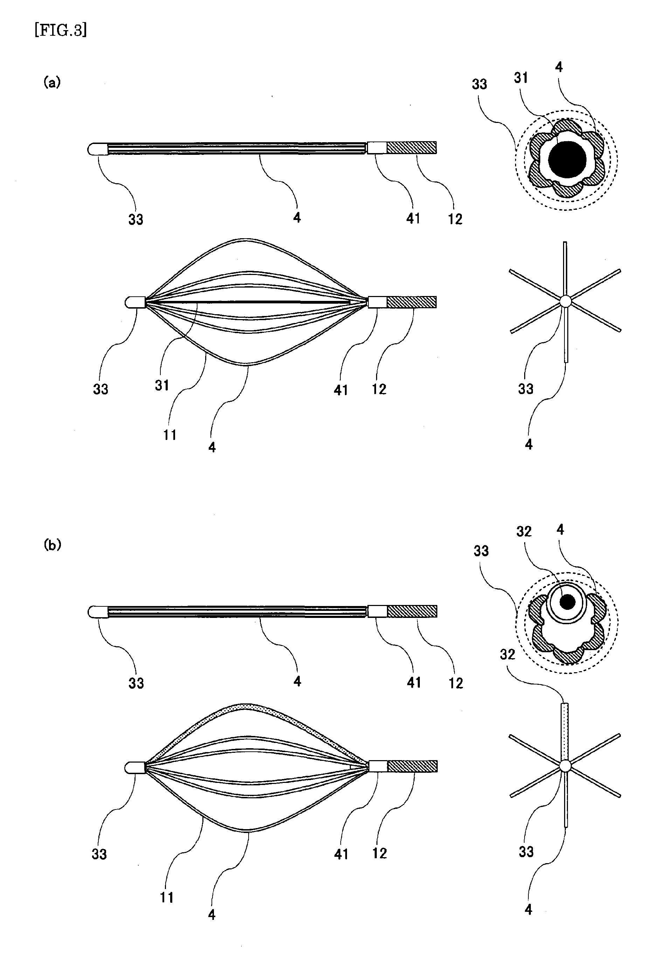

Referring to Figs. 2 and 3, the excluding portion 11 is configured by a movable wire 3 and a plurality of fixed wires 4. The movable wire 3 extends through the introduction portion, the proximal ends of the fixed wires 4 are fixed to the distal end of the introduction portion 12, and the distal end of the movable wire 3 and the distal ends of the fixed wires 4 are joined to each other. The fixed portion may be or may not be provided with a fixing member 41 that reinforces the fixture, and the joint portion may be or may not be provided with a joining member 33 that reinforces the joint. The number of movable wire 3 may be one or may be plural.

With the handle portion 13, the proximal end of the movable wire 3 is pulled toward the handle portion, and then the excluding portion 11 can develops a cocoon shape. The movable wire 3 may form a central axis of the developed cocoon shape, or may form one of the peripheral arcs of the cocoon shape.

In the case where the movable wire 3 forms a central axis of the cocoon shape (Fig. 3(a)), when the proximal end of the movable wire 3 is pulled toward the handle portion, the fixed wires 4 can develops a cocoon shape by forming curvature centered at the joint portion at the distal end. At that time, a pulling force toward the excluding portion is generated in the movable wire 3 because of the fixed wires 4 being curved. Accordingly, when the force that pulls the proximal end of the movable wire 3 toward the handle portion is relaxed, the development of the cocoon shape can be canceled. In this case, the fixed wires 4 in a number of preferably 4 to 8 form the peripheral arcs of the cocoon shape.

In the case where the movable wire 3 forms one of the peripheral arcs of the cocoon shape (Fig. 3(b)), for example, the movable wire 3 may be a "micro snake retractor" in which the movable wire 3 is made of ultra-thin high-strength twisted filamentary member having an outer diameter of 0.8 mm or less, and, in the excluding portion 11, is accommodated in a metal sheath connecting the joint portion at the distal end and the distal end of the introduction portion, having an outer diameter of 1.5 mm and being provided with a slit. For example, when the proximal end of the movable wire 3 is pulled toward the handle portion, the micro snake retractor forms a circular arc shape, and subsequently the fixed wires 4 form similar circular arc shapes centered at the joint portion at the distal end, and, thus, can develops a cocoon shape as a whole. In order to cancel the development of the cocoon shape, the excluding portion 11 is accommodated in the treatment instrument channel 22. In this case, there is no wire that forms the central axis of the cocoon shape, and the fixed wires 4 in a number of preferably 3 to 7 also form the peripheral arcs of the cocoon shape.

There is no particular limitation on the material for forming the movable wire 3, as long as it is rigid and straight enough to allow the entire movable wire 3 to move following the movement of the end portion, but the material is preferably metal. There is no particular limitation on the material for forming the fixed wires 4, as long as the movable wire 3 is rigid as appropriate, but the material is preferably metal. Examples of metal as the material for forming the movable wire 3 and the fixed wires 4 include stainless steel, tantalum, cobalt alloy, and nitinol (nickel-titanium alloy). Examples of stainless steel include SUS304, SUS316, and SUS316L. The wires may be in the shape of, for example, coils or wire meshes. The movable wire 3 and the fixed wires 4 each have a diameter of preferably 0.45 to 0.65 mm. The movable wire 3 and the fixed wires 4 may have the same diameter or may have different diameters, but preferably have the same diameter. Also, the fixed wires may have the same diameter or may have different diameters, but preferably have the same diameter.

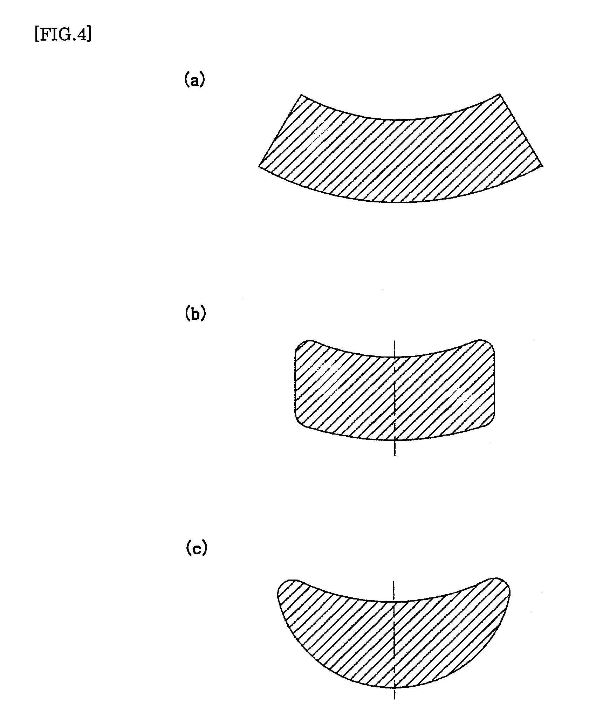

There is no particular limitation on the cross-sectional shape of the movable wire 3 that forms the central axis of the cocoon shape, but it is preferably a circular shape. Also, there is no particular limitation on the cross-sectional shape of the fixed wires 4 that form the peripheral arcs of the cocoon shape, but each of the fixed wire has preferably a shaped cross-section, more preferably a circular arc shaped cross-section, and even more preferably a crescent shaped cross-section (Figs. 3 and 4). The "shaped" herein refers to a particular shape different from a standard shape. The standard shape is a circular shape for the cross-section of a wire. Examples of the shaped cross-section include an elliptical shaped cross-section, a quadrangular shaped cross-section such as a rectangle shaped cross-section, and a triangular shaped cross-section. The shaped cross-section is preferably a circular arc shaped cross-section, and more preferably a crescent shaped cross-section. Figs. 4(a) and 4(b) show examples of the circular arc shaped cross-section, and Fig. 4(c) shows an example of the crescent shaped cross-section. It is particularly preferable that the center of each of the circular arc shapes (Figs. 4(a) and 4(b)) and an arc of the crescent shape (Fig. 4(c)) matches the central axis of the cocoon shape. With such a cross-sectional shape, when developing a cocoon shape, the fixed wires 4 can easily curved outward, the intervals between the wires developing the cocoon shape becomes an equal interval, and the rigidity of the cocoon shape increases. When not developing a cocoon shape, even in an embodiment having the central axis of the cocoon shape, the accommodability is high (upper right portion in Fig. 3(a): the cross-section of the excluding portion 11 when not developing a cocoon shape). There is no particular limitation on the major axis of each of the fixed wires 4 that form the peripheral arcs of the cocoon shape, but it is preferably 0.4 to 1.2 mm.

The excluding portion 11 is in an obtuse shape having an outer diameter of 2.5 mm or less when not developing a cocoon shape. The length may be set as appropriate according to the excluding operation or the shape of a target organ, but it is preferably 30 to 120 mm. The developed cocoon shape has an outer diameter of 20 to 70 mm. The outer diameter is typically 3 cm or less in the lumen of the gastrointestinal tract, and is typically 4 cm or more in the ordinary peritoneal cavity or in NOTES.

The number of wires that form the peripheral arcs of the cocoon shape is selected as appropriate according to the size of a cocoon shape that is to be developed, but it is typically 4 to 6 in the lumen of the gastrointestinal tract, and is typically 6 to 8 in the ordinary peritoneal cavity or in NOTES.

The developed cocoon shape has a wire interval that does not allow a target (organ) to enter the internal side, and a rigidity that allows the wires to freely exclude an organ. Furthermore, the cocoon shape can secure a space for manipulating a treatment instrument such as forceps, without obstructing the view of an endoscope.

Referring to Fig. 1, the introduction portion 12 extends from the excluding portion 11, and connects the handle portion 13 and the excluding portion 12. The length of the introduction portion 12 may be any length, as long as it is sufficient to cause the excluding portion 11 to be projected (or exposed) inside the body cavity. Typically, the length may be similar to that of a wire and the like extending from a treatment instrument for an endoscope. The introduction portion 12 can move inside the treatment instrument channel with the movement of the endoscope, without obstructing the movement of the endoscope.

The introduction portion 12 is in the shape of a hollow cylinder, and the movable wire 3 extends through the cylinder. There is no particular limitation on the material for forming the introduction portion 12, as long as it is flexible. Examples of the material for forming the introduction portion 12 include stainless steel such as SUS304, resin such as polyamide or PTFE, stainless steel coated with resin, and the like.

The excluding portion 11 and the introduction portion 12 have a smooth surface in order not to damage organs. The surface may be subjected to coating. The coating may employ materials commonly used for coating medical instruments. For example, the outer surface may be coated with porous polytetrafluoroethylene (ePTFE) film, silicone film, polyurethane film, polyethylene terephthalate (Dacron (registered trademark)) film, or the like.

Referring to Fig. 1, the handle portion 13 is provided at the proximal end of the introduction portion 12. With manipulation of the handle portion 13, the excluding portion 11 and the introduction portion 12 can be inserted into the treatment instrument channel of the endoscope, and the excluding portion 11 can be sent to the distal end of the endoscope and projected (or exposed) inside the body cavity.

The handle portion 13 can be manipulated so as to change the shape of the excluding portion 11. A method of changing the shape of the excluding portion 11 is, as described above, a method in which the proximal end of the movable wire 3 is pulled toward the handle portion.

There is no particular limitation on the shape and the structure of the handle portion 13, as long as the above-described functions can be realized. The size and the shape may be those allowing the instrument operator to easily manipulate the handle portion and commonly used in this technical field.

The retractor 1 of the present invention is inserted from the treatment instrument channel of the endoscope into a body cavity, and the excluding portion develops a cocoon shape. By manipulating the handle portion 13 or the endoscope while performing monitoring with the endoscope, the excluding portion 11 is brought into contact with the inner wall of a lumen, and is caused to press against or lift the inner wall, for example. With this manipulation, the operation of excluding, pushing aside, drawing, and lifting required on the outer wall of the lumen in intra-abdominal surgery and the like can be realized. Thus, appropriate traction can be realized on the outer wall of the hollow organ, and the view can be secured in the peritoneal cavity. Alternatively, for example, when the mucosal surface in the colon and rectum can be expanded and the folds are unfolded, the entire portion of a polyp hidden between the folds can be observed, and, when the stem thereof is exposed, procedures with an ordinary endoscope becomes possible.

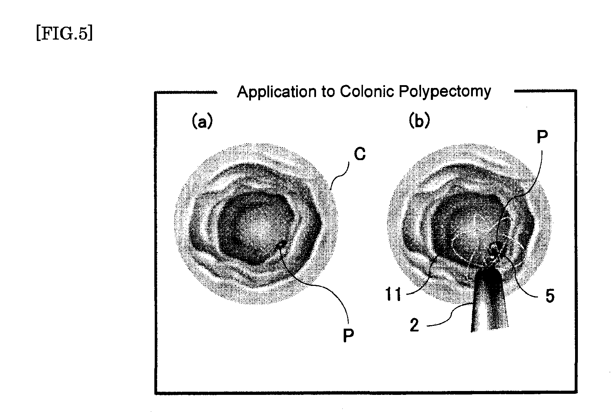

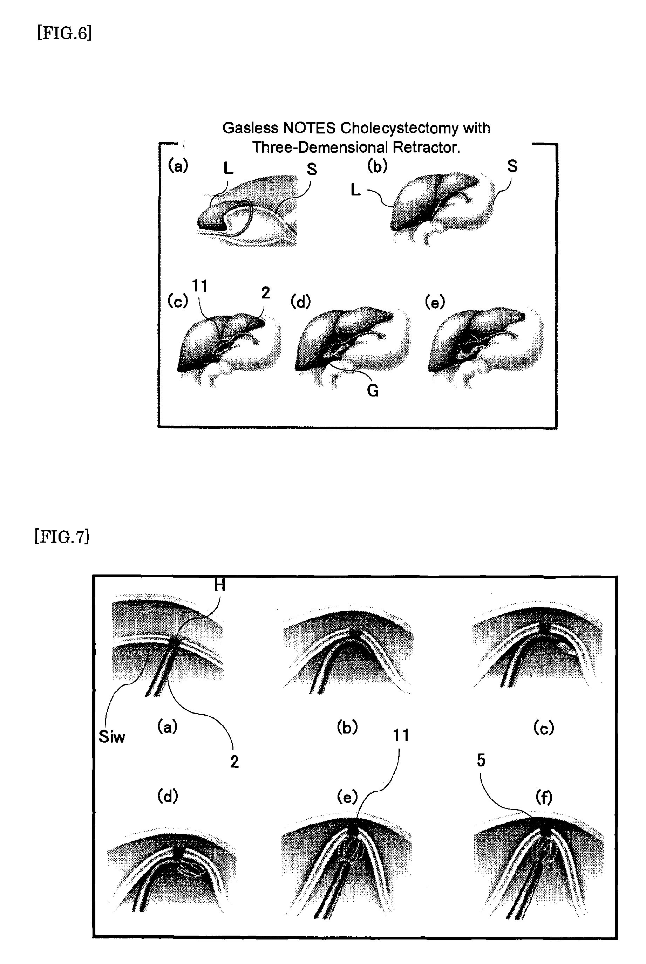

Hereinafter, the method of excluding an organ of the present invention will be specifically described by way of examples of application to colonic polypectomy (Fig. 5), application to cholecystectomy (Fig. 6), and application to closing of a small opening in the stomach in NOTES (Fig. 7).

Referring to Fig. 5, when performing resection of a polyp in the colon and rectum, the retractor 1 of the present invention is inserted into the treatment instrument channel of the endoscope 2, and is projected near a polyp P present in the colon and rectum C. In this example, the polyp P in the colon and rectum C is partially hidden between folds of the inner wall of the colon and rectum C, in a view from the endoscope 2 (Fig. 5(a)). Then, the retractor 1 of the present invention is arranged in the vicinity of the polyp P, and the excluding portion 11 developing a cocoon shape is pressed against the inner wall of the colon and rectum C in the vicinity of the polyp P to exclude the inner wall of the colon and rectum C such that the entire polyp P, in particular, its stem is visible (Fig. 5(b)). Since the view can be secured in this manner, for example, an electrocautery 5 inserted into the treatment instrument channel of the endoscope 2 and projected therefrom can reliably treat the polyp P, e.g., can coagulate the polyp P together with the blood vessels passing through the stem, can ligate and resect the polyp P, or the like.

Referring to Fig. 6, when performing resection of the gall bladder, a small opening is formed through the inner wall of the stomach S, and the endoscope 2 is inserted from the small opening through the inner wall of the stomach S into the peritoneal cavity (Fig. 6(a)). The retractor 1 of the present invention is inserted into the treatment instrument channel of the endoscope 2, and is projected near the gall bladder G that is present under the liver L (Fig. 6(b)). In this example, the gall bladder G is covered by the liver L in a view from the endoscope 2, and is invisible unless the liver L is pushed aside. Then, the retractor 1 of the present invention is arranged in the vicinity of the gall bladder G, and is caused to develop a cocoon shape (Fig. 6(c)). Then, the excluding portion 11 developing the cocoon shape is pressed below the liver L to lift the liver L such that the entire gall bladder G is visible (Fig. 6(d)). Since the view can be secured in this manner, for example, an electrocautery inserted into the treatment instrument channel of the endoscope 2 and projected therefrom can reliably resect the gall bladder G (Fig. 6(e)).

Referring to Fig. 7, in NOTES, a small opening H is formed through the stomach's inner wall Siw, and the endoscope 2 is inserted from the small opening H into the peritoneal cavity, and, then, procedures are performed. After the procedures, it is necessary to return endoscope 2 back to the stomach, and close the small opening H endoscopically. In conventional methods, an attempt is made to secure the view by injecting gas into the stomach, but, actually, the gas leaks out from the small opening H into the peritoneal cavity, and the stomach collapses, so that a good view cannot be secured. In this case, the retractor 1 of the present invention is inserted into the treatment instrument channel of the endoscope 2, and is caused to develop a cocoon shape near the small opening H to be closed in the stomach, so that the view and the operation space necessary for closing the small opening H can be secured, without injecting gas (Figs. 7(a) to 7(e)). Accordingly, it is possible to reliably close the small opening H formed in the stomach, using a small opening closing device 5 that has been inserted into the treatment instrument channel of the endoscope 2 and projected therefrom (Fig. 7(f)). The method of the present invention is effective also in the unexpected case of piercing through the wall of the intestinal tract during endoscopic therapy.

### [Industrial Applicability]

The present invention can provide a three-dimensional retractor that can freely exclude an organ, and can be inserted into a treatment instrument channel of an endoscope. The retractor of the present invention is inserted via a treatment instrument channel of an endoscope into the lumen of a hollow organ or into a body cavity, and quickly develops a cocoon shape with remote manipulation. The developed cocoon shape makes it possible to freely exclude organs, and, thus, this retractor is useful, for example, in minimally-invasive treatment such as NOTES.

The method of the present invention can secure the view and the manipulation space without injecting gas into a body cavity (gassless surgery), and, thus, general anesthesia is not necessary, and very minimally-invasive endoscopic surgery becomes possible.

More specifically, in transvaginal NOTES, the retractor of the present invention can be applied via a gastroscope to gastrectomy, esophagectomy, small bowel resection, splenectomy (where the stomach is expanded from the lumen for the expansion between the stomach and the spleen), and the like. In transgastric NOTES, the retractor can be applied via an enteroscope or a colonscope to colectomy, proctectomy, small bowel resection, and the like. Alternatively, the retractor can be also applied to the fields of gynecology, urology, respiratory surgery, and the like.

Furthermore, the retractor of the present invention can be applied not only to NOTES but also to laparoscopic surgery, endoscopic surgery, and laparotomy surgery. For example, in order to realize appropriate traction on the gastrointestinal tract in ordinary laparoscopic surgery, the retractor can be used by being inserted via an endoscope into the lumen of the gastrointestinal tract. Such manipulation eliminates the need of an organ retracting member to be used laparoscopically, and, thus, can reduce incisional wounds in the abdominal region.

Furthermore, the retractor of the present invention can be used to assist in complicated manipulation using an ordinary gastrointestinal endoscope (gastroscope or colonscope). For example, it is possible to cause a polyp that is present on the back side of folds, which is difficult to access, to be apparent on the front by unfolding the wall using the retractor of the present invention, and resect it endoscopically.

### [References Signs List]

- 1: retractor
- 11: excluding portion
- 12: introduction portion
- 13: handle portion
- 2: endoscope
- 21: objective optical system (camera lens)
- 22: treatment instrument channels
- 3: movable wire
- 31: one embodiment of the movable wire
- 32: one embodiment of the movable wire
- 33: joining member
- 4: fixed wires
- 41: fixing member
- 5: device for surgery manipulation (an electrocautery, a small opening closing device etc.)
- C: colon and rectum
- P: polyp
- S: stomach
- Siw: stomach's inner wall
- L: liver
- G: gall bladder
- H: small opening

## Claims

1. A retractor comprising an excluding portion having an obtuse distal end, an introduction portion extending from the excluding portion, and a handle portion provided at a proximal end of the introduction portion,
wherein the excluding portion and the introduction portion each have an outer diameter that allows insertion into a treatment instrument channel of an endoscope,
the excluding portion is configured by a movable wire and a plurality of fixed wires,
the movable wire extends through the introduction portion,
proximal ends of the fixed wires are fixed to a distal end of the introduction portion,
a distal end of the movable wire and distal ends of the fixed wires are joined to each other, and
with the handle portion, a proximal end of the movable wire is pulled toward the handle portion so that the excluding portion can develop a cocoon shape.

2. The retractor of claim 1, wherein the movable wire forms a central axis of the cocoon shape.

3. The retractor of claim 1, wherein the movable wire forms one of the peripheral arcs of the cocoon shape.

4. The retractor of any one of claims 1 to 3, wherein each of the fixed wires that form the peripheral arcs of the cocoon shape has a shaped cross-section.

5. The retractor of any one of claims 1 to 4, wherein each of the fixed wires that form the peripheral arcs of the cocoon shape has a circular arc shaped cross-section.

6. The retractor of any one of claims 1 to 5, wherein each of the fixed wires that form the peripheral arcs of the cocoon shape has a crescent shaped cross-section.

7. The retractor of any one of claims 1 to 6, wherein the excluding portion has a length of 30 to 120 mm.

8. The retractor of any one of claims 1 to 7, wherein each of the fixed wires that form the peripheral arcs of the cocoon shape has a cross-section having a major axis of 0.4 to 1.2 mm.

9. A method of excluding an organ, which comprises:
causing an endoscope provided with a treatment instrument channel to be inserted from a natural orifice of a hollow organ into the lumen, or through a wall of the lumen into a body cavity;
inserting the retractor of any one of claims 1 to 8 into the treatment instrument channel;
causing the excluding portion of the retractor to project into the lumen or the body cavity so as to develop a cocoon shape; and
bringing the excluding portion into contact with an inner wall of the hollow organ or an organ in the body cavity to exclude the inner wall or the organ.

10. The method of claim 9, wherein the method is performed in surgery selected from the group consisting of laparoscopic surgery and NOTES.

11. A method of securing a view in a surgery in a lumen or a surgery in a body cavity, which comprises:
causing an endoscope provided with a treatment instrument channel to be inserted from a natural orifice of a hollow organ into the lumen, or through a wall of the lumen into a body cavity;
inserting the retractor of any one of claims 1 to 8 into the treatment instrument channel;
causing the excluding portion of the retractor to project into the lumen or the body cavity so as to develop a cocoon shape near an inner wall of the hollow organ or an organ in the body cavity, which obstructs the view; and
bringing the excluding portion into contact with the inner wall or the organ, to exclude the inner wall or the organ, thereby securing the view in the lumen or the body cavity.

12. The method of claim 11, wherein the surgery in the body cavity is selected from the group consisting of laparoscopic surgery and NOTES.
